# EUROPEAN PATENT SPECIFICATION

(11) **EP 2 165 651 B1**
(45) Date of publication and mention of the grant of the patent: **17.08.2011**
(21) Application number: 08164745.5
(22) Date of filing: 19.09.2008
(51) Int. Cl.: A61B 17/02

(54) **Surgical tool for vascular exposure and access**
Chirurgisches Werkzeug für Gefäßfreilegung und -zugang
Outil chirurgical pour exposer et accéder aux vaisseaux sanguins

(43) Date of publication of application: 24.03.2010
(73) Proprietor: SORIN BIOMEDICA CARDIO S.R.L., 13040 Saluggia (Vercelli) (IT)
(72) Inventor: Manasse, Eric, 20123, Milano (IT); Vallana, Franco, 10123, Torino (IT)
(74) Representative: Bosotti, Luciano

(56) References cited:
- EP-A- 1 210 912
- WO-A-99/38440
- WO-A-2007/017294
- US-A- 5 213 114
- US-A- 6 136 017
- US-A1- 2002 095 166

## Description

### TECHNICAL FIELD

The present invention relates to surgical instruments, and more particularly, to surgical instruments for use in prosthetic heart valve procedures and other vascular procedures requiring access to the inside of a vessel or organ.

### BACKGROUND

Conventional surgical heart valve repair and/or replacement procedures generally require complete dissection of the ascending aorta in order to suture the replacement valve (e.g., a prosthetic aortic valve) to the native valve annulus. With surgical sutureless heart valve replacement procedures, however, complete dissection of the ascending aorta would not be necessary for suturing the valve in place (i.e., because no suturing is required), but is still generally required to gain access to the native valve and valve annulus and generally to the inside of the vessel. More specifically, the invention relates to a tool for surgery according to the preamble of claim 1, which is known, e.g., from WO 2007/017294A. Documents such as e.g., WO 99/38440A, EP1210912A or US 5213114A are also of interest for the invention.

### SUMMARY

The present invention, having the features at forth in claim 1, is a tool for facilitating exposure of an inside portion of a vessel or an organ, the vessel having a proximal end and a distal end. The tool comprises a substantially cylindrical band having a deployed configuration capable of opening the proximal portion of the vessel and an arm connected to the band. The arm has a deployed configuration capable of at least partially flattening the distal end of the vessel.

The present invention, refers to a tool for surgery facilitating exposure of an inside section of a vessel. The tool comprises an element having a band capable of creating an operating field to the inside of a first portion of the vessel and an arm capable of at least partially flattening a second portion of the vessel. According to some embodiments, the element is sized and dimensioned so that a prosthetic heart valve can travel therethrough. In some embodiments, the element is made from a lightweight material, such as a biocompatible polymer. According to some embodiments, the outside of at least a portion of the element is textured so as not to tear the intima of the vessel. In some embodiments, the band has a deployed configuration capable of exerting an outward radial force on the vessel wall, the outward radial force of the element being sufficient to adapt to different inner diameters of the vessel, while avoiding destructive stretching of the inner wall of the vessel.

According to some embodiment, the arm comprises a bar having a deployed configuration generally perpendicular to a plane of the band. The bar may have an exemplary length of from about 3 cm to about 5 cm. The arm may have a generally t-shaped deployed configuration.

The tools of the present invention, are adapted for use in a method of surgery comprising, partially dissecting a vessel to create an opening of a dissected vessel, inserting a tool through the opening, and exposing an operating field that is located within the vessel or organ connected to the vessel through the use of the tool. The method of surgery, which do not form part of the invention, may further comprise providing a valve prosthesis and passing the prosthesis through the opening made by the tool, wherein the prosthesis may be a valve prosthesis.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 is a perspective partial cut-away view of a surgical tool in a deployed state according to one embodiment of the present invention.

FIGS. 2A-2C are schematic elevation, plan, and end views, respectively, of the surgical tool of FIG. 1.

FIGS. 3A and 3B are plan views schematically illustrating the surgical tool of FIG. 1 partially and fully deployed in a patient's ascending aorta.

FIG. 4 is an end view schematically illustrating the surgical tool of FIG. 1 deployed in a patient's ascending aorta taken along the line 4-4 of FIG. 3B.

FIGS. 5A and 5B are elevation and side schematic views of a portion of an alternative surgical tool according to another embodiment of the present invention.

### DETAILED DESCRIPTION

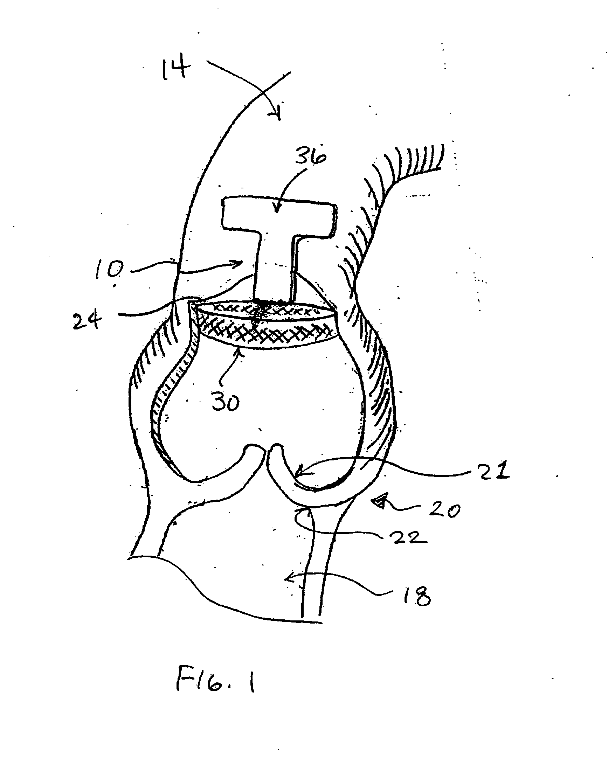

FIG. 1 is a perspective partial cut-away view of a surgical tool 10 in a deployed state within a patient's ascending aorta 14 according to one embodiment of the present invention. As is known, and as shown in FIG. 1, the ascending aorta 14 is coupled to the left ventricle 18. During normal operation, the left ventricle 18 pumps blood out of the heart through the aortic valve 20 and into the ascending aorta 14. The aortic valve 20 is a semilunar valve including a set of valve leaflets 21 surrounding an aortic annulus 22, which is defined by the periannular tissue located at the most distal portion of the left ventricular outflow tract.

In the illustrated embodiment, the surgical tool 10 is deployed through a partial incision 24 in the wall of the ascending aorta 14 in a manner as would be used, for example, to facilitate surgical procedures in the vicinity of the aortic valve. Exemplary procedures that may be performed in conjunction with the surgical tool 10 include, without limitation, repair of the aortic valve 20, replacement of the aortic valve 20, aortic annuloplasty, repair procedures in the left ventricle, and similar procedures. In one embodiment, the surgical tool 10 is advantageously utilized for implantation of a surgical sutureless prosthetic heart valve to replace the aortic valve 20. As explained in further detail below, the surgical tool 10 operates, at least in part, to provide an open operating field through which the surgeon or other clinician can perform the particular procedure. While much of the following disclosure is directed to use of the surgical tool 10 for use in procedures relating to the aortic valve 20, one of ordinary skill in the art will readily recognize that the tool 10 will have utility for a wide variety of other procedures requiring access into various portions of the vasculature system or organs.

In the illustrated embodiment, the surgical tool 10 includes a band 30 and an arm 36 coupled thereto. As shown, the band 30 is sized to be inserted into the ascending aorta 14 proximal to the incision 24 (i.e., closer to the left ventricle), and the arm 36 extends distally (i.e., farther from the left ventricle) from the band 30 and across the incision 24. In FIG. 1, for illustration purposes, the wall of the ascending aorta 14 proximal to the incision 24 is shown cut-away to better illustrate the position of the band 30. In practice, the band 30 is inserted into the ascending aorta 14 proximal to the incision 24 and is allowed to expand into contact with the internal surface (i.e., intima) of the vessel. The band 30 is configured to hold open and support the wall of the that portion of the ascending aorta 14 (or other desired vessel) with sufficient radial force to substantially inhibit migration of the band 30 from a desired location near the incision 24. The band 30 thus provides a relatively large operating field through which the physician can access and visualize the aortic valve 20, the valve leaflets 21, and the aortic annulus 22.

As shown in FIG. 1, the arm 36 extends distally across the incision 24. As shown, however, the arm 36 is not inserted into the ascending aorta 14. Rather, the arm 36 contacts the outside anterior wall of the ascending aorta 14 and urges this anterior wall toward the posterior wall so as to somewhat flatten the ascending aorta 14 distal to the incision 24. This flattening of the portion of the ascending aorta 14 distal to the incision 24 (which would otherwise interfere or block the operating lumen within the aorta 14 proximal to the incision 24) further enlarges the operating field available to the physician. Thus, the surgical tool 10 operates to maximize the operating field available to the physician while requiring only a partial incision about the circumference of the ascending aorta 14 (i.e., without requiring complete dissection of the ascending aorta 14).

While the above discussion refers to the arm 36 urging the anterior wall of the ascending aorta 14 toward the posterior wall thereof, the use of the surgical tool 10 is not limited to only an anterior/posterior circumferential orientation. That is, the surgical tool 10 can be oriented so as to locate the arm 36 anywhere about the circumference of the ascending aorta 14. Additionally, although the arm 36 will typically be positioned generally diametrically opposite the center of the incision 24, this is not a requirement.

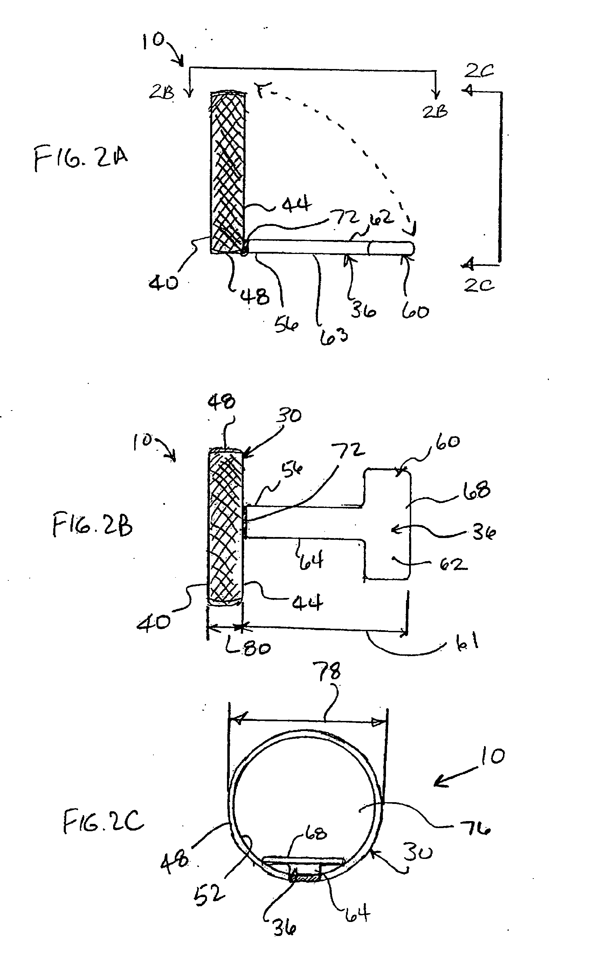

FIGS. 2A-2C are schematic elevation, plan, and end views, respectively, of the surgical tool 10 according to one embodiment of the present invention. As shown in FIGS. 2A-2C, in the illustrated embodiment, the band 30 is generally cylindrical and defines a proximal edge 40, a distal edge 44, an outer face 48, and an inner face 52.

As further shown, the arm 36 has a proximal end 56 and a distal end 60 defining a length 61, an upper face 62, and a lower face 63, and includes a lateral member 64 and a cross member 68. Additionally, in the illustrated embodiment, the proximal end 56 of the arm 36 is coupled to the band 30 by a hinge 72 located at or proximate the distal edge 44 of the band 30. In the illustrated embodiment, the lateral member 64 extends away from the hinge 72, and the cross member 68 is located on the lateral member 64 opposite the hinge 72, such that the distal end 60 of the arm 36 is generally T-shaped.

As illustrated in FIG. 2A, the arm 36 is angularly adjustable with respect to the band 30 by virtue of the hinge 72. Thus, the arm 36 is pivotable or actuatable between a longitudinally compact configuration (not shown), in which the arm 36 is substantially parallel to the general plane of the band 30, and the deployed configuration of FIGS. 2A-2C in which the arm 36 extends upward and is substantially perpendicular to the general plane of the band 30. The hinge 72 can take on any suitable configuration. In various embodiments, the hinge 72 is a separate structure attached to both the band 30 and the proximal end 56 of the arm. In other embodiments, the hinge 72 is integral to one or both of the band 30 and the arm 36. In various embodiments, the hinge 72 is in the form of a so-called "living hinge," which structures are well known in the art and need not be described in detail here.

In various embodiments, the arm 36 is pre-configured to be biased toward the deployed configuration of FIGS. 2A-2C, and temporarily retained in the compact configuration by any suitable retaining structures (e.g., sutures). In such embodiments, once the band 30 is placed in the proximal ascending aorta 14 as desired by the physician, the retaining structures can be removed or otherwise disabled (e.g., the sutures can be cut) so as to allow the arm 36 to rotate to its deployed configuration, thereby flattening the ascending aorta 14 distal to the incision 24. In other embodiments, the arm 36 is manually rotatable or adjustable by the physician.

As will be appreciated, the arm 36 is configured such that all or a substantial portion of the lower face 63 will contact and bear upon the outer surface of the ascending aorta 14 distal to the incision 24 when deployed. In various embodiments, all or a portion of the lower face 63 is textured to help minimize damage to the vessel wall. In other embodiments, the lower face 63 is not textured. Because the upper face 62 is not generally configured to contact the wall of the aorta, in various embodiments, this face is generally smooth (i.e., not textured). Of course, the upper face 62 can be textured as well if desired.

In the illustrated embodiment, the arm 36 is shown as a generally unitary structure. This is not a requirement, however, and in other embodiments, the arm 36 can be formed from one or more wires, rods, etc. into the desired shape. Of course, the arm 36 need not be T-shaped as shown. Additionally, the arm 36 need not be substantially flat as shown. Rather, in various embodiments, all or portions of the arm 36 can have other cross-sectional shapes, e.g., cylindrical. In some exemplary embodiments, the cross member 68 is formed with a slight curvature such that it is concave from the perspective of the center of the vessel.

As further shown, the band 30 is generally cylindrical, with the inner face 52 defining an opening 76, the outer face 48 defining an outer diameter 78, with the separation between the outer and inner faces defining a width 80. The outer diameter 78 is generally selected such that the outer face 48 contacts and bears upon the intima of the ascending aorta 14 in which the band is inserted. Such contact is selected to be sufficient to prevent or at least substantially inhibit translation of the band 30 within the desired vessel (e.g., ascending aorta 14), and also to provide sufficient support to maintain the desired operating field, but at the same time to avoid unnecessary stretching of the wall of the vessel. The width 80 is also selected to optimize the contact area between the outer face 48 and the intima of the ascending aorta 14 without interfering with the implantation of the valve prosthesis. In exemplary embodiments, the width 80 is between about 1 and about 15 mm. In other exemplary embodiments, the width 80 is between about 5 and about 10 mm.

Similar to the lower face 63 of the arm 36 described above, the band 30 is configured such that all or a substantial portion of the outer face 48 will contact and bear upon the intima of the ascending aorta 14 proximal to the incision 24 when deployed. In various embodiments, all or a portion of the outer face 48 is textured to help minimize damage to the vessel wall. In other embodiments, the outer face 48 is not textured. Because the inner face 52 is not generally configured to contact the intima of the aorta, in various embodiments, this face is generally smooth (i.e., not textured). Of course, the inner face 52 can be textured as well if desired.

In one embodiment, the band 30 is radially expandable from a radially compact configuration (not shown) suitable for ease of deployment, to a radially expanded, deployed configuration such as shown in FIGS. 2A-2C. In such an embodiment, the outer diameter 78, when the band 30 is radially un-restrained, is larger than the inner diameter of the corresponding portion of the ascending aorta 14 in which the band 30 will be inserted. Thus, upon insertion into the ascending aorta 14 and subsequent expansion, the band 30 will exert an outward radial force on the inner wall of the ascending aorta 14. The band 30 is configured such that the applied radial force is sufficient to allow the band to adapt to a range of inner aortic diameters, but not to the extent that the ascending aortic wall is unnecessarily stretched.

In various embodiments, the expandable band 30 may be self-expanding, in which case the band 30 is pre-biased to its expanded configuration, and is retained in its radially compact configuration prior to insertion into the ascending aorta 14 via a suitable retaining structure. After insertion into the ascending aorta 14, the retaining structure is removed or disable, thereby allowing the band 30 to attempt to expand to its un-restrained outer diameter 78. Alternatively, the band 30 may be manually expandable by another device, e.g., a balloon catheter. Various technologies and configurations for self-expanding and/or balloon-expandable structures are known, such as, for example, technologies used in the design and manufacture of stents for interventional cardiology procedures, which technologies could be utilized in the design and manufacture of the band 30.

The overall dimensions of the band 30 and the arm 36 can be tailored to provide the desired functionality (i.e., support of the ascending aorta 14 proximal to the incision and flattening of the ascending aorta 14 distal to the incision). As will be appreciated, the outer diameter 78 is selected based on the size of the ascending aorta 14 in which it is to be inserted. Additionally, the opening 76 is generally sized to maximize the operating field available to the physician. As will be appreciated, the size of the opening 76 is dictated by the outer diameter 78 and the thickness of the material making up the band 30. In various embodiments, the band 30 is made from a lightweight material having sufficient strength to provide the desired support, but still allowing the thickness of the band 30 to be minimized, thereby maximizing the size of the opening 76. In various embodiments, the opening 76 is sized to permit passage of a prosthetic heart valve (e.g., a surgical sutureless prosthetic aortic valve) therethrough, along with other instruments necessary for implantation of the prosthetic valve.

The T-shaped profile of the arm 36 advantageously allows the width of the lateral member 64 to be minimized, so as to not unnecessarily stiffen the band 30 at the attachment (e.g., the hinge 72) between the two structures, yet still provide significant contact between the arm 36 and the wall of the ascending aorta 14. Additionally, the length 61 of the arm 36 can be varied as needed. According to some embodiments, the length of the arm 36 is approximately equal to the diameter of the band 30 when the band is in its compressed form. In various embodiments, the length 61 is selected in the range of from about 1 cm to about 5 cm. In other embodiments, the length 61 is selected in the range of from about 3 cm to about 5 cm. In another variant of the invention the arm could also have a concave or convex profile as viewed from above, and or the top part of the T could have the same characteristics.

The band 30 and/or arm 36 may be made from any material having suitable physical characteristics. In various embodiments, the band 30 and/or the arm 36 are made from a lightweight, biocompatible polymeric or metallic material. In embodiments where the band 30 is expandable, at least the band 30 is made from a biocompatible polymer or metal having shape memory and/or super-elastic properties. One such class of shape-memory and super-elastic materials are nickel-titanium alloys such as nitinol. In various embodiments, for example, where the arm 36 is self-deployable, the hinge 72 may also be made from stainless steel or a shape-memory material such as nitinol.

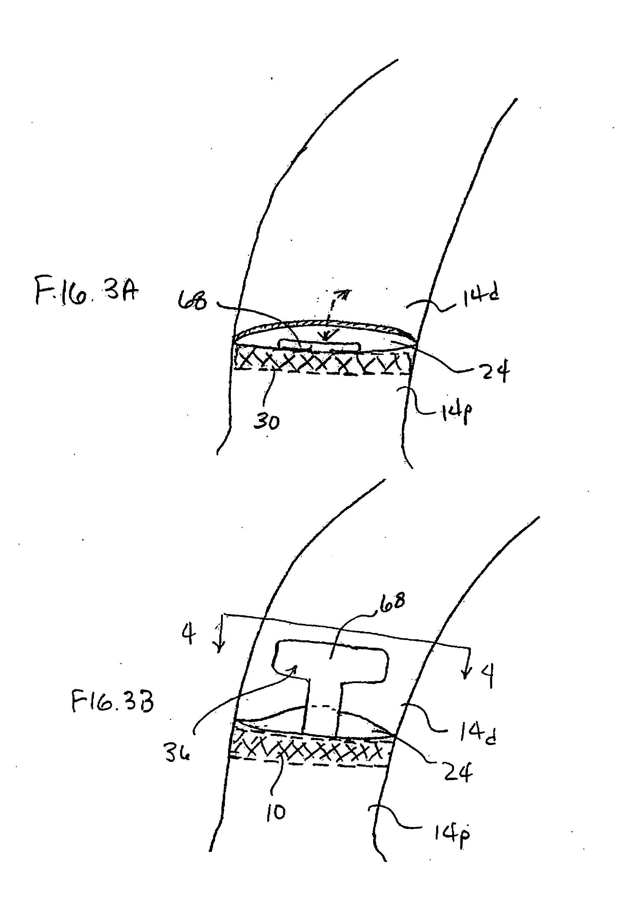

FIGS. 3A and 3B are plan views schematically illustrating the surgical tool 10 partially and fully deployed, respectively, in a patient's ascending aorta. In FIG. 3A, the surgical tool 10 is partially deployed, in that the band 30 is inserted through the incision 24 into the proximal ascending aorta 14p, and the arm 36 is as yet undeployed, such that the cross-member 68 is positioned adjacent to the band 30. In FIG. 3B, the arm 36 has been deployed (i.e., angularly adjusted to displace the cross-member 68 away from the band 30), thereby at least partially flattening the distal ascending aorta 14d. As illustrated in FIGS. 3A and 3B, the surgical tool 10 is amenable to implantation by the physician through a relatively small aortic incision. The incision 24 need only be of a length sufficient to allow insertion of the surgical tool 10, while in its compressed configuration. The incision 24 is made through about half of the diameter of the aortic wall.

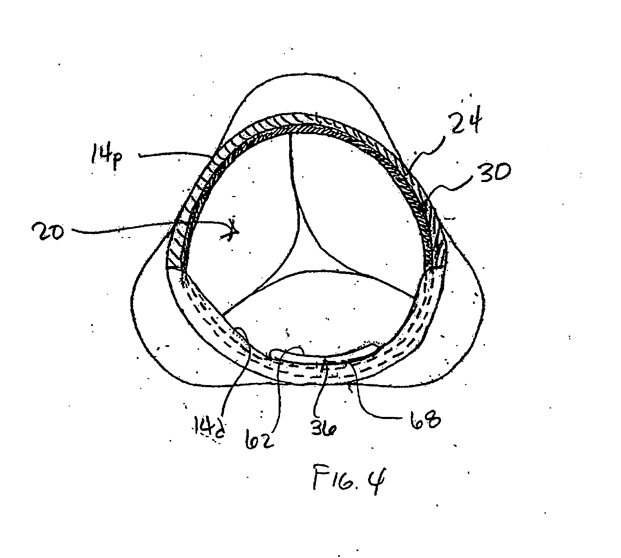

FIG. 4 is an end view schematically illustrating the surgical tool 10 deployed in a patient's ascending aorta taken along the line 4-4 of FIG. 3B. As shown in FIG. 4, in the deployed state, the band 30 contacts and supports the proximal ascending aorta 14p, while the deployed arm 36 has substantially flattened the distal ascending aorta 14d. Thus, the operating field available to the physician (e.g., for visualizing and accessing the diseased aortic valve 20) is maximized while still allowing the length of the incision 24 to be relatively short (as compared to a complete aortic dissection). Additionally, in the embodiment illustrated in FIG. 4, the arm 36, including the cross-member 68, is curved in the same general shape as the shape of the ascending aorta 14 so as to further reduce the extent to which the arm 36 extends radially into the operating field. In one embodiment, the arm 36 is pre-shaped to generally correspond to the aortic anatomy. In other embodiments, all or portions of the arm 36, e.g., the cross-member 68, are flexible so as to adapt or conform to the anatomy as desired.

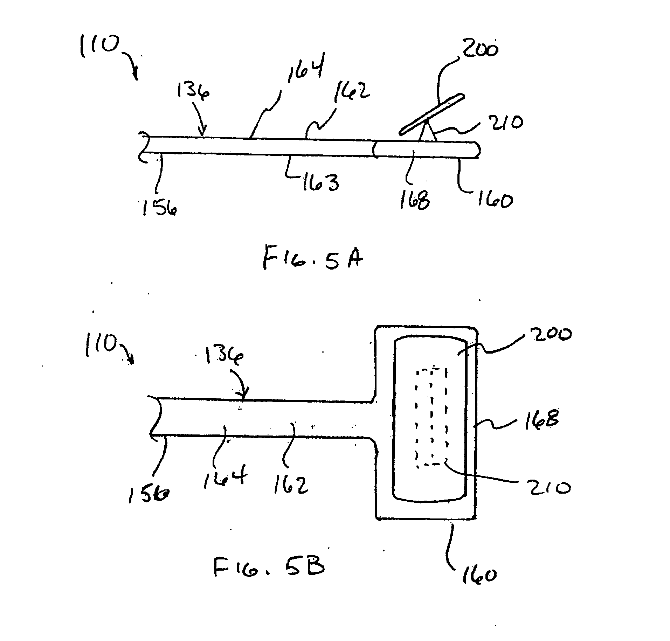

FIGS. 5A and 5B are elevation and side schematic views of a portion of an alternative surgical tool 110 according to another embodiment of the present invention. As shown in FIGS. 5A and 5B, the surgical tool 110 includes a arm 136 which is configured to be coupled to a band such as the band 30 of the tool 10 discussed above. The arm 136 is overall configured in substantially the same or an identical manner as the arm 36 of the tool 10. As such, the arm 136 has a proximal end 156 and a distal end 160, an upper face 162, and a lower face 163, and includes a lateral member 164 and a cross member 168. In the illustrated embodiment, the cross member 168 is located on the lateral member 164 opposite the proximal end 156, such that the distal end 160 of the arm 136 is generally T-shaped.

Additionally, the arm 136 includes a mirror 200 pivotally coupled to a standoff 210. As shown, the standoff is attached to and supported from the upper face 162 of the cross-member 168. The mirror 200 can pivot about the attachment point to the standoff 210, and can be oriented to provide enhanced visualization of the inside of the aorta and/or the operating field in general.

Various modifications and additions can be made to the exemplary embodiments discussed without departing from the scope of the present invention. Accordingly, the scope of the present invention is intended to embrace all such alternatives, modifications, and variations as fall within the scope of the appended claims.

## Claims

1. A tool (10) for surgery facilitating exposure of an inside section of a vessel comprising an element having a band (30) sized to be inserted into the vessel and allowed to expand into contact with the internal surface of the vessel in a deployed configuration such as to exert a radial force to hold open and support the vessel for creating an operating field to the inside of a first portion of the vessel and an arm (36) coupled to the band (30), **characterized in that** the arm (36) is rotatably coupled to the band (30) and wherein in the rotated deployed configuration the arm (36) extends distally from the band (30) and across an incision in said vessel for at least partially flattening a second portion of the vessel.

2. The tool of claim 1 wherein the element (30, 36) is sized and dimensioned so that a prosthetic heart valve can travel therethrough.

3. The tool of claim 2 wherein the prosthetic heart valve is a surgical sutureless heart valve.

4. The tool of claim 1 whereby the element (30, 36) comprises a lightweight material.

5. The tool of claim 4 wherein the lightweight material is selected from the group consisting of: a disposable material, a sterile material, and a biocompatible material.

6. The tool of claim 4 whereby the lightweight material has a low profile to avoid reducing the lumen of the vessel.

7. The tool of claim 1 wherein the width of the band (30) is between about 5 and about 10 mm.

8. The tool of claim 1 wherein the outside (48, 63) of at least a portion of the element (30, 36) is textured so as not to tear the intima of the vessel.

9. The tool of claim 1 wherein the band (30) has a deployed configuration capable of exerting an outward radial force on the vessel wall, the outward radial force of the element being sufficient to adapt to different inner diameters of the vessel, while avoiding destructive stretching of the inner wall of the vessel.

10. The tool of claim 1 wherein the element (30, 36) is self-expanding and/or outwardly biased.

11. The tool of claim 1 wherein the arm (36) comprises a bar having a deployed configuration generally perpendicular to a plane of the band.

12. The tool of claim 11 wherein the bar has a length of from about 3 cm to about 5 cm.

13. The tool of claim 1 wherein the arm (36) has a generally t-shaped deployed configuration.

14. The tool of claim 1 or claim 13 wherein the arm (36) is angularly adjustable with respect to the band (30), preferably by a hinge (72) pivotably coupling the arm (36) to the band (30).

15. The tool of claim 1 wherein at least a portion (48, 63) of the element (30, 36) that contacts the vessel is textured, while an inner portion (52) of the band (30) is substantially smooth.

16. The tool of claim 1 or claim 13 wherein the arm (36) comprises a mirror (200).

17. The tool of claim 16 wherein the mirror (200) is adjustable or pivotable, whereby the mirror permits additional visualization of the inside of the vessel.

18. The tool of claim 13 wherein the generally t-shaped arm (36) comprises a top portion (68), the top portion (68) being slightly curved, whereby the top portion (68) substantially adapts to the anatomy of the vessel while avoiding conflicts in a surgical field.

## Patentansprüche

1. Chirurgisches Werkzeug (10), welches die Freilegung des Inneren eines Gefäßes ermöglicht, umfassend ein Element mit einem Band (30), das dimensioniert ist, um in das Gefäß eingeführt werden und in Kontakt mit der inneren Oberfläche des Gefäßes in eine ausgefahrene Konfiguration expandieren zu können, so dass eine radiale Kraft ausgeübt wird, um das Gefäß offen zu halten und zu unterstützen und so ein Operationsfeld zu dem Inneren eines ersten Bereiches des Gefäßes zu erzeugen, und einem Arm (36) der mit dem Band (30) verbunden ist, **dadurch gekennzeichnet, dass** der Arm (36) rotierbar mit dem Band (30) verbunden ist, und dass sich in der rotierten ausgefahrenen Konfiguration der Arm (36) distal von dem Band (30) und über einen Schlitz in dem Gefäß erstreckt, um wenigstens teilweise einen zweiten Bereich des Gefäßes abzuflachen.

2. Chirurgisches Werkzeug nach Anspruch 1, **dadurch gekennzeichnet, dass** das Element (30, 36) derart bemessen und dimensioniert ist, dass eine Herzklappenprothese hindurchlaufen kann.

3. Chirurgisches Werkzeug nach Anspruch 2, **dadurch gekennzeichnet, dass** die Herzklappenprothese eine chirurgische nahtlose Herzklappe ist.

4. Chirurgisches Werkzeug nach Anspruch 2, **dadurch gekennzeichnet, dass** der Bestandteil (30, 36) ein Leichtbaumaterial umfasst.

5. Chirurgisches Werkzeug nach Anspruch 4, **dadurch gekennzeichnet, dass** das Leichtbaumaterial ausgewählt ist aus der Gruppe bestehend aus: einem Wegwerfmaterial, einem sterilen Material und einem biokompatiblen Material.

6. Chirurgisches Werkzeug nach Anspruch 4, **dadurch gekennzeichnet, dass** das Leichtbaumaterial einen niedrigen Querschnitt aufweist, um zu verhindern, dass das Lumen des Gefäßes reduziert wird.

7. Chirurgisches Werkzeug nach Anspruch 1, **dadurch gekennzeichnet, dass** die Breite des Bandes (30) zwischen etwa 5 und etwa 10 mm beträgt.

8. Chirurgisches Werkzeug nach Anspruch 1, **dadurch gekennzeichnet, dass** die Außenseite (48, 63) von zumindest einem Bereich des Elementes (30, 36) derart strukturiert ist, dass die Intima des Gefäßes nicht zerrissen wird.

9. Chirurgisches Werkzeug nach Anspruch 1, **dadurch gekennzeichnet, dass** das Band (30) eine ausgefahrene Konfiguration aufweist, in der eine nach außen gerichtete radiale Kraft auf die Gefäßwand ausgeübt wird, wobei die nach außen gerichtete radiale Kraft des Elemetes ausreichend ist, um sich an verschiedene innere Durchmesser des Gefäßes anzupassen, während eine schädigende Dehnung der Innenwand des Gefäßes verhindert wird.

10. Chirurgisches Werkzeug nach Anspruch 1, **dadurch gekennzeichnet, dass** das Element (30, 36) selbstexpandierend und/oder nach außen vorgespannt ist.

11. Chirurgisches Werkzeug nach Anspruch 1, **dadurch gekennzeichnet, dass** der Arm (36) einen Stab umfasst, der eine ausgefahrene Konfiguration aufweist, die im allgemeinen senkrecht zu der Ebene des Bandes ist.

12. Chirurgisches Werkzeug nach Anspruch 11, **dadurch gekennzeichnet, dass** der Stab eine Länge von etwa 3 cm bis etwa 5 cm aufweist.

13. Chirurgisches Werkzeug nach Anspruch 1, **dadurch gekennzeichnet, dass** der Arm (36) eine im Allgemeinen T-förmige ausgefahrene Konfiguration aufweist.

14. Chirurgisches Werkzeug nach Anspruch 1 oder Anspruch 13, **dadurch gekennzeichnet, dass** der Arm (36) in seinem Winkel relativ zu dem Band (30) einstellbar ist, vorzugsweise durch ein Scharnier (72), das den Arm (36) schwenkbar mit dem Band (30) verbindet.

15. Chirurgisches Werkzeug nach Anspruch 1, **dadurch gekennzeichnet, dass** wenigstens ein Bereich (48, 63) des Elementes (30, 36), der das Gefäß berührt, texturiert ist, während ein innerer Bereich (52) des Bandes (30) im Wesentlichen glatt ist.

16. Chirurgisches Werkzeug nach Anspruch 1 oder 13, **dadurch gekennzeichnet, dass** der Arm (36) einen Spiegel (200) umfasst.

17. Chirurgisches Werkzeug nach Anspruch 16, **dadurch gekennzeichnet, dass** der Spiegel (200) einstellbar oder schwenkbar ist, wobei der Spiegel eine zusätzliche Visualisierung des Inneren des Gefäßes ermöglicht.

18. Chirurgisches Werkzeug nach Anspruch 13, **dadurch gekennzeichnet, dass** der im Allgemeinen T-förmige Arm (36) einen oberen Bereich (68) aufweist, wobei der obere Bereich (68) leicht gebogen ist, und der obere Bereich (68) sich im Wesentlichen an die Anatomie des Gefäßes anpasst, während Konflikte in einem operativen Gebiet vermieden werden.

## Revendications

1. Instrument (10) de chirurgie facilitant l'exposition d'une section interne d'un vaisseau comprenant un élément ayant une bande (30) dimensionnée pour être insérée dans le vaisseau et autorisée à s'expanser en contact avec la surface interne du vaisseau dans une configuration déployée pour exercer une force radiale afin de maintenir ouvert et supporter le vaisseau pour créer une champ opératoire à l'intérieur d'une première partie du vaisseau et un bras (36) couplé à la bande (30),
**caractérisé en ce que** le bras (36) est couplé de manière rotative à la bande (30) et dans lequel, dans la configuration déployée entraînée en rotation, le bras (36) s'étend de manière distale à partir de la bande (30) et d'un côté à l'autre d'une incision dans ledit vaisseau pour aplatir au moins partiellement une deuxième partie du vaisseau.

2. Instrument selon la revendication 1, dans lequel l'élément (30, 36) est taillé et dimensionné de sorte qu'une valvule cardiaque prothétique puisse se déplacer à travers ce dernier.

3. Instrument selon la revendication 2, dans lequel la valvule cardiaque prothétique est une valvule cardiaque chirurgicale sans suture.

4. Instrument selon la revendication 1, moyennant quoi l'élément (30, 36) comprend un matériau léger.

5. Instrument selon la revendication 4, dans lequel le matériau léger est choisi dans le groupe comprenant un matériau jetable, un matériau stérile et un matériau biocompatible.

6. Instrument selon la revendication 4, moyennant quoi le matériau léger a un profil bas pour éviter de réduire la lumière du vaisseau.

7. Instrument selon la revendication 1, dans lequel la largeur de la bande (30) est comprise entre environ 5 et environ 10 mm.

8. Instrument selon la revendication 1, dans lequel l'extérieur (48, 63) d'au moins une partie de l'élément (30, 36) est texturisée afin de ne pas déchirer l'intima du vaisseau.

9. Instrument selon la revendication 1, dans lequel la bande (30) a une configuration déployée pouvant exercer une force radiale vers l'extérieur sur la paroi du vaisseau, la force radiale vers l'extérieur de l'élément étant suffisante pour s'adapter aux différents diamètres internes du vaisseau, tout en évitant l'étirement destructeur de la paroi interne du vaisseau.

10. Instrument selon la revendication 1, dans lequel l'élément (30, 36) est autoexpansible et/ou sollicité vers l'extérieur.

11. Instrument selon la revendication 1, dans lequel le bras (36) comprend une barre ayant une configuration déployée généralement perpendiculaire à un plan de la bande.

12. Instrument selon la revendication 11, dans lequel la barre a une longueur de l'ordre d'environ 3 cm à environ 5 cm.

13. Instrument selon la revendication 1, dans lequel le bras (36) a une configuration déployée généralement en forme de t.

14. Instrument selon la revendication 1 ou la revendication 13, dans lequel le bras (36) peut être ajusté de manière angulaire par rapport à la bande (30), de préférence par une charnière (72) couplant le bras (36) de manière pivotante à la bande (30).

15. Instrument selon la revendication 1, dans lequel au moins une partie (48, 63) de l'élément (30, 36) qui est en contact avec le vaisseau est texturisée, alors qu'une partie interne (52) de la bande (30) est sensiblement lisse.

16. Instrument selon la revendication 1 ou la revendication 13, dans lequel le bras (36) comprend un miroir (200).

17. Instrument selon la revendication 16, dans lequel le miroir (200) peut être ajusté ou pivoté, moyennant quoi le miroir permet la visualisation supplémentaire de l'intérieur du vaisseau.

18. Instrument selon la revendication 13, dans lequel le bras (36) généralement en forme de t comprend une partie supérieure (68), la partie supérieure (68) étant légèrement courbée, moyennant quoi la partie supérieure (68) s'adapte sensiblement à l'anatomie du vaisseau tout en évitant les conflits dans un champ opératoire.
